**Europäisches Patentamt**

**European Patent Office**

**Office européen des brevets**

(19)

(11) Veröffentlichungsnummer : **0 093 251**
**B1**

(12)

# EUROPÄISCHE PATENTSCHRIFT

(45) Veröffentlichungstag der Patentschrift :
**12.11.86**

(51) Int. Cl.⁴ : **A 61 L 9/12**

(21) Anmeldenummer : **83102580.4**

(22) Anmeldetag : **16.03.83**

(54) **Raumodorant.**

(30) Priorität : **03.05.82 DEU 8212730**
**11.09.82 DEU 8225646**

(43) Veröffentlichungstag der Anmeldung :
**09.11.83 Patentblatt 83/45**

(45) Bekanntmachung des Hinweises auf die Patenterteilung : **12.11.86 Patentblatt 86/46**

(84) Benannte Vertragsstaaten :
**BE CH DE FR GB IT LI LU NL**

(56) Entgegenhaltungen :
**FR-A- 418 288**
**US-A- 1 989 883**
**US-A- 4 293 095**

(73) Patentinhaber : **Azur Fragrances France S.A.**
**Mas St. Mathieu Chemin Peyloubet**
**F-06130 Grasse (FR)**

(72) Erfinder : **Freytag von Loringhoven, Andreas**
**Mas.St.Mathieu Chemin Peyloubet**
**F-06130 Grasse (FR)**

(74) Vertreter : **Sturies, Herbert et al**
**Patentanwälte Dr. Ing. Dipl. Phys. Herbert Sturies**
**Dipl. Ing. Peter Eichler Postfach 20 12 42**
**D-5600 Wuppertal 2 (DE)**

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

## Beschreibung

Die Erfindung betrifft eine Kunstblume mit einem in ihrem Kelch versteckt und auswechselbar angeordneten Duftstoffträger aus mit Duftstoff getränktem, saugfähigem Werkstoff.

Bei einer durch die FR-A-418 288 bekannten Kunstblume obiger Art besteht der Duftstoffträger aus einem Watte-Stopfen, der unmittelbar vor einer im Kelch der Blume angeordneten Sprühdüse einer Duftstoff-Zerstäubereinrichtung angeordnet ist, die als flüssigen Duftstoff beinhaltender Vorratsbehälter zusammen mit einem als Betätigungsorgan dienenden Handballon in einer die Kunstblume aufnehmenden Vase untergebracht ist und deren Duftstoff über einen schlauchartig ausgebildeten Blütenstengel der Kunstblume der Sprühdüse zugeführt werden kann. Es handelt sich hier also um ein in einer Kunstblumen-Vase untergebrachtes Pumpenspraysystem, an dessen im Blumenkelch gelegenen Ausgang sich der das ausgespritzte Parfüm aufnehmende Watte-Stopfen als Duftstoffträger befindet. Eine solche duftabgebende Kunstblume ist als Raumodorant, der in seiner Umgebung einen angenehmen Duft verbreiten, insbesondere die Raum- oder Zimmerluft wohlriechend machen soll, sehr umständlich zu handhaben und auch äußerst aufwendig, allein schon im Hinblick auf die Anordnung und Unterbringung ihrer Zerstäubereinrichtung in der Blumen-Vase.

Der Erfindung liegt demgegenüber die Aufgabe zugrunde, eine duftabgebende Kunstblume zu schaffen, die hinsichtlich ihres Duftstoffträgers wesentlich einfacher beschaffen ist, kostensparender hergestellt werden kann und ohne besonderes Duftstoffpumpsystem auskommt.

Diese Aufgabe wird erfindungsgemäß dadurch gelöst, daß der Duftstoffträger als aus Vliesstoff bestehender, zylindrischer Dufttampon ausgebildet ist, der an seinem einen Ende mit einer fest auf ihm angebrachten, aus Kunststoff bestehenden Befestigungskappe versehen und auf einem deren Boden durchdringenden Dorn eines im Blumenkelch untergebrachten Untersatzes aufgespießt ist. Der äußerst einfach beschaffene und leicht herzustellende Dufttampon ist infolge der absorbierenden Fähigkeiten seines Faservliesstoffes vorzüglich zum Aufbringen oder als Träger von Duftstoffen geeignet. Er wird damit gleichzeitig zum Duftstoffreservoir und zur Duftstoffquelle. Die an seinem einen Ende vorhandene, auf dem Dorn des Untersatzes aufzuspießende Befestigungskappe aus Kunststoff ermöglicht seine stabile Fixierung im Blumenkelch. Darüber hinaus kann der Dufttampon bei Nachlassen oder Erschöpfung seiner Duftstoffabgabe leicht ausgewechselt und durch einen neuen Dufttampon ersetzt werden. Dabei versteht es sich, daß der im Dufttampon gespeicherte Duftstoff bzw. dessen Aroma der Art der damit auszustattenden Kunstblume tunlichst entsprechen sollte, wobei auch die Farbe des Dufttampons der Kunstblume entsprechend gewählt werden kann.

In der Zeichnung ist ein Ausführungsbeispiel einer nach der Erfindung gestalteten Kunstblume dargestellt, und zwar in

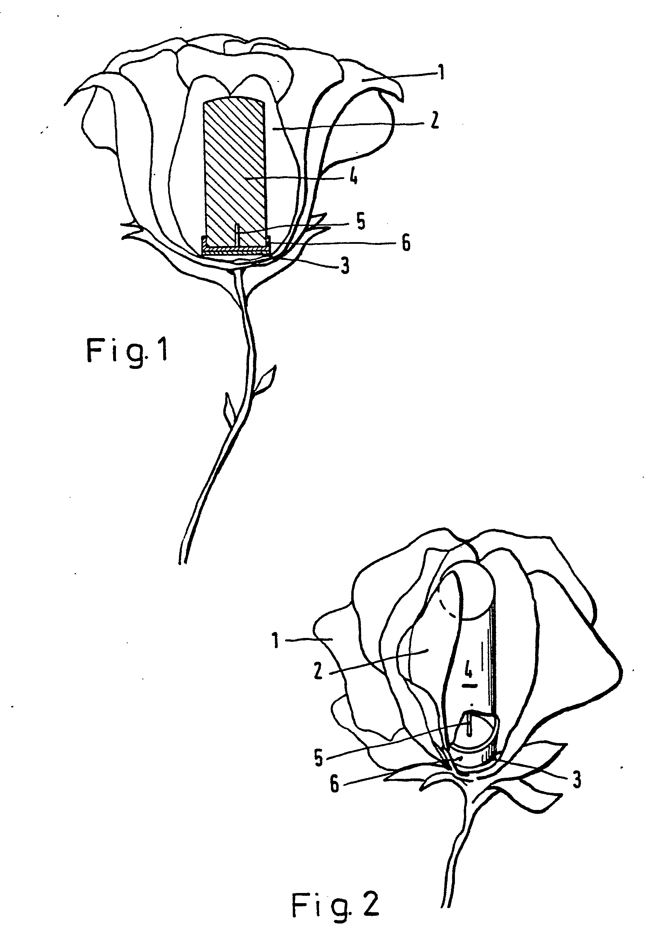

Figur 1  in teilweisem Längsschnitt und

Figur 2  in der Draufsicht bei teilweise entfernten Kelchblättern.

Die abgebildete Kunstblume 1 ist in ihrem Kelch 2 mit einer kleinen runden Scheibe 3 versehen, die als Untersatz für den darauf anzubringenden Duftstoffträger 4 dient. Dieser ist als aus Faservliesstoff bestehender, tamponartiger Duftdocht ausgebildet. Zum Befestigen des Dufttampons 4 ist auf der den Untersatz bildenden Scheibe 3 ein aufrecht stehender Stift bzw. Dorn 5 vorgesehen, auf dem der Dufttampon 4 ohne Schwierigkeiten klemmend aufzuspießen ist. Da das Material des Dufttampons 4 im Sinne einer starken Saugfähigkeit sehr weich ausgebildet ist und um ein dadurch bedingtes Abrutschen des Dufttampons 4 vom Dorn 5 mit Sicherheit zu verhindern, ist der Dufttampon 4 an seinem unteren Ende mit einer fest an ihm angebrachten, aus Kunststoff bestehenden Befestigungskappe 6 versehen, deren Boden von dem Dorn 5 des scheibenförmigen Untersatzes 3 durchdrungen wird.

Der zylindrische Dufttampon 4 kann je nach Art der nachzubildenden Blume 1 eine Länge von beispielsweise 1 bis 4 cm und einen Durchmesser bis zu etwa 1,5 cm haben sowie der Blume entsprechend eingefärbt sein. In einem Ausführungsbeispiel war der Dufttampon 4 je nach Umgebungstemperatur ca. vier bis sechs Wochen wirksam. Er wurde dann zusammen mit seiner Befestigungskappe 6 aus der Kunstblume 1 entfernt und durch einen mit frischem Duftkonzentrat beaufschlagten Dufttampon ersetzt.

## Patentanspruch

Kunstblume mit einem in ihrem Kelch versteckt und auswechselbar angeordneten Duftstoffträger aus mit Duftstoff getränktem, saugfähigem Fasermaterial, dadurch gekennzeichnet, daß der Duftstoffträger als aus Vliesstoff bestehender zylindrischer Dufttampon (4) ausgebildet ist, der an seinem einen Ende mit einer fest auf ihm angebrachten, aus Kunststoff bestehenden Befestigungskappe (6) versehen und auf einem deren Boden durchdringenden Dorn (5) eines im Blumenkelch (2) untergebrachten Untersatzes (3) aufgespießt ist.

## Claim

An artificial flower having a replaceable fragrance-carrying element of absorbent fibrous material impregnated with an aromatic substance hidden in its calyx, characterised in that the fragrance-carrying element is formed as a cylindrical fragrant plug (4) of a fleecy material which is provided at its one end with a plastics fixing cap (6) fixedly mounted thereon and which is penetrated through the base of the cap by a spike (5) of a support (3) accommodated in the flower calyx (2).

**Revendication**

Fleur artificielle munie d'un support interchangeable de substance odorante, dissimulé dans sa corolle et réalisé en une matière fibreuse absor-

:

bante, imprégnée de substance odorante, caractérisée en ce que le support de substance odorante est réalisé sous la forme d'un tampon parfumé cylindrique (4) en une matière textile qui est pourvu, à l'une de ses extrémités, d'une calotte de fixation (6) en matière plastique dont il est fermement solidaire, et qui est planté sur une pointe (5) d'un élément de soutien (3) logé dans la corolle (2) de la fleur, cette pointe transperçant le fond de la calotte de fixation.

Fig. 1

Fig. 2

1